# EUROPÄISCHE PATENTSCHRIFT

(11) **EP 1 843 813 B1**
(45) Veröffentlichungstag und Bekanntmachung des Hinweises auf die Patenterteilung: **27.08.2008**
(21) Anmeldenummer: 05819969.6
(22) Anmeldetag: 23.12.2005
(51) Int. Cl.: A61M 39/28

(54) **SCHLAUCHKLEMME**
HOSE CLAMP
PINCE POUR TUYAUX SOUPLES

(30) Priorität: 02.02.2005 DE 102005004863
(43) Veröffentlichungstag der Anmeldung: 17.10.2007
(73) Patentinhaber: Fresenius Medical Care Deutschland GmbH, 61352 Bad Homburg (DE)
(72) Erfinder: RUFFING, Andreas, 66450 Bexbach (DE)
(74) Vertreter: Laufhütte, Dieter
(86) Internationale Anmeldenummer: PCT/EP2005/013969
(87) Internationale Veröffentlichungsnummer: WO 2006/081866

(56) Entgegenhaltungen:
- EP-A- 0 995 461
- WO-A-20/04041343
- DE-U1- 9 011 416

## Beschreibung

Die Erfindung betrifft eine Schlauchklemme, vorzugsweise zum Abklemmen von Katheterverlängerungen, nach dem Oberbegriff des Anspruchs 1.

Derartige Schlauchklemmen sind bereits bekannt. So beschreibt beispielsweise die EP 0 995 461 A1 eine Schlauchklemme, die aus einem sich länglich erstreckenden einteiligen Kunststoffkörper mit zwei einseitig miteinander verbundenen Armen besteht. Die beiden Armen bilden auf ihrer Innenseite jeweils einen Vorsprung derart aus, dass sich die Vorsprünge im geschlossenen Zustand der Schlauchklemme gegenüber liegen und so dimensioniert sind, dass sie den Schlauch abdrücken. Beide Arme sind im geschlossenen Zustand miteinander verhakbar. An ihrem vorderen und hinteren Ende weist die Schlauchklemme jeweils Öffnungen auf, über die ein Schlauch, beispielsweise eine Katheterverlängerung, aufnehmbar ist.

Derartige Schlauchklemmen werden beispielsweise auf dem Gebiet der Peritonealdialyse an Katheteterverlängerungen verwendet. Dabei befindet sich die Klemme am Bauchanschluss des Patienten und dient zum Öffnen und Verschließen der am Bauchanschluss befindlichen Katheterverlängerung. Da die Klemme dauerhaft an der Haut des Patienten anliegt, kommt es bei der vorbekannten Klemme und bei anderen handelsüblichen Klemmen, die für diese Verwendung grundsätzlich geeignet sind, oftmals zu Hautreizungen wegen der vorhandenen Grate und Kanten. Auch Größe und Form werden vom Patienten als störend empfunden.

Die Aufgabe der vorliegenden Erfindung besteht darin, die vorbekannten Schlauchklemmen derart weiterzubilden, dass sie vom Patienten beim Tragen nicht mehr als störend empfunden werden.

Erfindungsgemäß wird die Aufgabe durch eine Schlauchklemme mit einer Merkmalskombination gemäß dem Patentanspruch 1 gelöst. Demnach weist die Schlauchklemme nahezu die Form eines Zylinders mit abgeschnittenen Seiten auf, wobei sich dieser beidseitig zu seinen Enden hin konisch verjüngt und wobei die Enden an die kreisrunde Form eines einzulegenden Schlauches angepaßt sind. Die Idee dieser neuen Formgebung der Schlauchklemme liegt darin, dass die Grundform von einem Zylinder abgeleitet ist und dass die Enden konisch zulaufend ausgebildet sind. Um einen möglichst großen Tragekomfort zu ermöglichen, ist die Form der Schlauchklemme an die Form des Schlauches angepaßt.

Vorteilhafte Ausgestaltungen der Erfindung ergeben sich aus den sich an den Hauptanspruch anschließenden Unteransprüchen. Demnach sind sämtliche Kantenbereiche der Schlauchklemme abgerundet, so dass scharfe Kanten und Ecken vermieden werden.

Gemäß einer bevorzugten Ausführungsform übersteigt die Länge der Schlauchklemme nicht den zehnfachen Schlauchdurchmesser. Weiterhin ist es vorteilhaft, dass die Radien des abgerundeten Kunststoffkörpers zwischen R20 und R100 liegen.

Weiterhin ist es vorteilhaft, dass der Kunststoffkörper zu seinen Enden hin unter einem Winkel von 5 Grad bis 40 Grad zur Längsachse ausläuft. Hierdurch wird ein kontinuierlicher Übergang von dem Schlauch zur Klemme hin möglich. Gemäß einer anderen bevorzugten Ausgestaltung liegt der äußerste Punkt der Schlauchklemme im geschlossenen Zustand maximal um den doppelten Schlauchdurchmesser von der Mitte des einzulegenden Schlauches entfernt weg. Hierdurch wird eine schlanke Form der Schlauchklemme definiert, die vom Patienten nicht mehr störend empfunden wird.

Zur leichteren Handhabbarkeit sind an der ansonsten im wesentlichen zylindrischen Außenfläche der beiden Arme Griffflächen ausgenommen.

Vorteilhaft ist der Verhakmechanismus einerseits durch einen zum freien Ende des Armes weisenden Vorsprung am Ende der Schlauchklemme und andererseits durch eine am freien Ende des Armes ausgebildete Anlagekante gebildet. Dabei ist oberhalb des Vorsprungs am freien Ende der Schlauchklemme eine Grifffläche ausgebildet, über die das elastische Ende der Schlauchklemme aus der Verrastposition in eine Freigabeposition auslenkbar ist. Hierdurch ist gewährleistet, dass die Schlauchklemme mehrfach geschlossen und geöffnet werden kann.

Weitere Einzelheiten und Vorteile der Erfindung ergeben sich anhand den in der beigefügten Zeichnungen dargestellten Ausführungsbeispielen. Es zeigen:
- Figur 1:: eine Seitenansicht einer erfindungsgemäßen Schlauchklemme,
- Figur 2:: die Seitenansicht gemäß Figur 1 mit einer eingezeichneten Schnittlinie A-A,
- Figur 3:: den Schnitt A-A gemäß Figur 2,
- Figur 4:: eine Ansicht von vorne auf eine Schlauchklemme gemäß Figur 1,
- Figur 5:: eine Draufsicht von oben auf eine Schlauchklemme gemäß Figur 1 und
- Figur 6:: ein Schnitt B-B gemäß Figur 5.

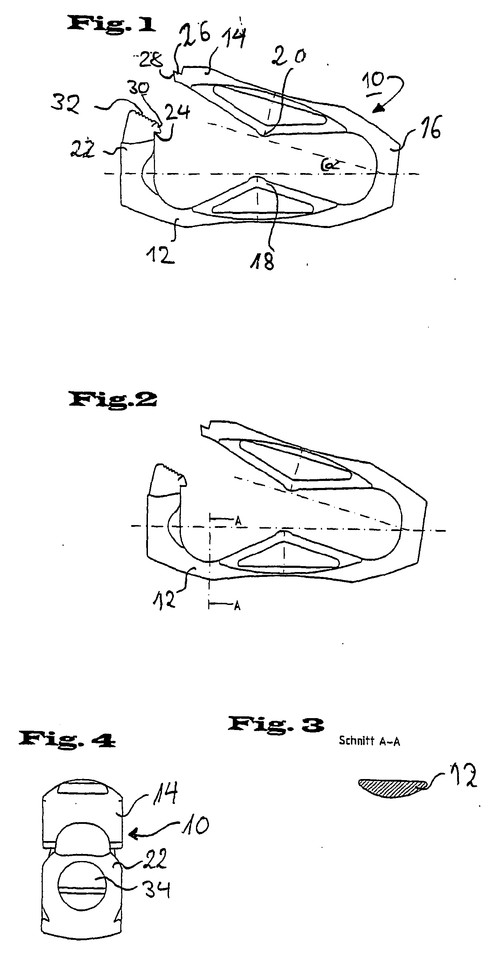

In der Figur 1 ist eine Schlauchklemme 10 gemäß einer Ausführungsform der vorliegenden Erfindung gezeigt. Die Schlauchklemme 10 besteht aus einem sich länglich erstreckenden einteiligen Kunststoffkörper der zwei Arme 12 und 14 aufweist. Die Arme sind an einem Ende 16 der Schlauchklemme miteinander verbunden, wobei sie im unbelasteten Zustand derart geformt sind, dass der Arm 14 gegenüber dem Arm 12 um den Winkel α ausgelenkt ist. Die Arme 12 und 14 weisen jeweils Vorsprünge 18 und 20 auf. Mittels dieser Vorsprünge kann ein Schlauchstück, auf das die Schlauchklemme aufgefädelt ist, abgeklemmt werden. Gegen die Federkraft des Endteils 16 kann der Arm 14 in Richtung zum Arm 12 verschwenkt werden und im geschlossenen Zustand mit dem Endteil 22 verhakt werden. Der Verhakmechanismus ist einerseits durch ein zum freien Ende des Armes weisenden Vorsprung 24 am Endteil der Schlauchklemme 10 und andererseits eine am freien Ende des Arms ausgebildete Anlagekante 26 gebildet. Am vorderen Ende der Anlagekante 26 und am entsprechenden Ende des Vorsprungs 24 sind jeweils sich entsprechende Schrägen 28 und 30 angeordnet, die dazu führen, dass beim Niederdrücken des Arms 14 das Ende 22 ausgelenkt wird, so dass die Anlagekante 26 unterhalb des Vorsprungs 24 zu liegen kommt, so dass die Schlauchklemme im geschlossenen Zustand verhakt wird.

Am freien Ende der Schlauchklemme ist zusätzlich eine Grifffläche 32 ausgebildet, über die das elastische Ende 22 der Schlauchklemme aus der Verrastposition in eine Freigabeposition auslenkbar ist.

Wie insbesondere aus der Figur 4 zu sehen, sind im Bereich des Vorderteils 22 aber auch, wie hier nicht näher dargestellt, im Hinterteil 16 jeweils kreisrunde Öffnungen 34 vorgesehen, durch die der Schlauch oder Katheter in die Schlauchklemme eingeführt wird.

Die Schlauchklemme 10 weist eine nahezu der Form eines Zylinders mit abgeschnittenen Seiten angenäherte Form aus. Zu beiden Enden 16 und 22 hin verjüngt sich der Zylinder konisch und die jeweiligen Enden 16 und 22 weisen jeweils eine kreisrunde Form auf, die derjenigen des einzulegenden Schlauches angepaßt ist. Sämtliche Kantenbereiche in der Schlauchklemme sind hier abgerundet, wie sich beispielsweise aus der Schnittdarstellung A-A des Arms 12 gemäß Figur 3 ergibt.

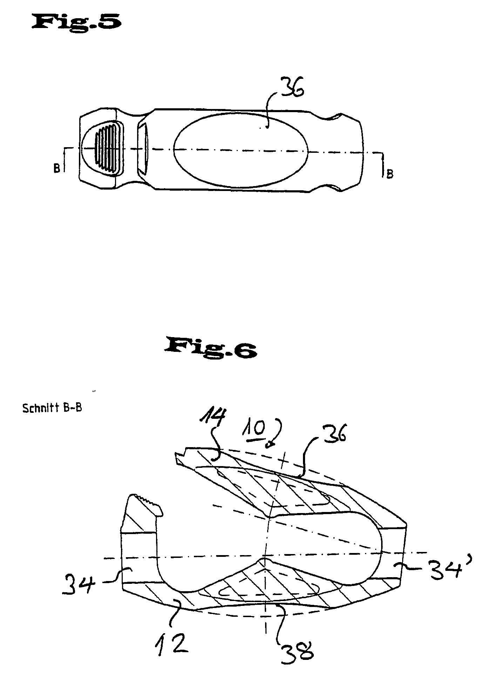

Wie insbesondere aus den Figuren 5 und 6 zu ersehen, sind an den Armen 12 und 14 jeweils am Umfang Abflachungen als Grifffläche 36 und 38 ausgebildet, die eine einfachere Handhabbarkeit ermöglichen.

## Patentansprüche

1. Schlauchklemme (10), vorzugsweise zum Abklemmen von Katheterverlängerungen, bestehend aus einem sich länglich erstreckenden einteiligen Kunststoff-körper mit zwei einseitig federnd miteinander verbundenen Armen (12,14), die jeweils einen Vorsprung (24,26) derart bilden, daß diese sich im geschlossenen Zustand der Schlauchklemme gegenüberliegen, und die im geschlossenen Zustand mit-einander verhakbar sind, wobei Öffnungen (34,34') am vorderen und hinteren Ende jeweils zur Aufnahme eines Schlauches vorgesehen sind,
**dadurch gekennzeichnet,**
**daß** sie nahezu die Form eines Zylinders mit abgeschnittenen Seiten aufweist, der sich beidseitig zu seinen Enden hin konisch verjüngt und daß die Enden an die kreisrunde Form eines einzulegenden Schlauches angepaßt sind.

2. Schlauchklemme nach Anspruch 1, **dadurch gekennzeichnet, daß** sämtliche Kantenbereiche abgerundet sind.

3. Schlauchklemme nach Anspruch 1 oder 2, **dadurch gekennzeichnet, daß** ihre Länge nicht den 10-fachen Schlauchdurchmesser übersteigt.

4. Schlauchklemme nach einem der Ansprüche 1 bis 3, **dadurch gekennzeichnet, daß** die Radien des abgerundeten Kunststoffkörpers zwischen R20 und R100 liegen.

5. Schlauchklemme nach einem der Ansprüche 1 bis 4, **dadurch gekennzeichnet, daß** der Kunststoffkörper zu seinen Enden hin unter einem Winkel von 5° bis 40° zur Längsachse ausläuft.

6. Schlauchklemme nach einem der Ansprüche 1 bis 5, **dadurch gekennzeichnet, daß** ihr äußerster Punkt im geschlossenen Zustand maximal den doppelten Schlauchdurchmesser von der Mitte des einzulegenden Schlauches entfernt liegt.

7. Schlauchklemme nach einem der Ansprüche 1 bis 6, **dadurch gekennzeichnet, daß** an der Außenfläche der beiden Arme Griffflächen ausgenommen sind.

8. Schlauchklemme nach einem der Ansprüche 1 bis 7, **dadurch gekennzeichnet, daß** der Verhakmechanismus einerseits durch einen zum freien Ende des Armes weisenden Vorsprung am Ende der Schlauchklemme und andererseits eine am freien Ende des Armes ausgebildete Anlagekante gebildet wird.

9. Schlauchklemme nach Anspruch 8, **dadurch gekennzeichnet, daß** oberhalb des Vorsprunges am freien Ende der Schlauchklemme eine Grifffläche ausgebildet ist, über die das elastische Ende der Schlauchklemme aus der Verrastposition in eine Freigabeposition auslenkbar ist.

## Claims

1. A hose clamp (10), preferably for clamping off catheter extensions, comprising an elongate one-piece plastic body with two arms (12, 14) which are resiliently connected together at one end and which each form a respective projection (24, 26) in such a way that they are in opposite relationship in the closed condition of the hose clamp and are hookable to each other in the closed condition, wherein openings (34, 34') are provided at the front and rear ends respectively for receiving a hose,
**characterised in that**
it is almost in the form of a cylinder with cut-off sides, which tapers conically at both sides towards its ends and that the ends are adapted to the circular shape of a hose to be inserted.

2. A hose clamp according to claim 1 **characterised in that** all edge regions are rounded off.

3. A hose clamp according to claim 1 or claim 2 **characterised in that** the length thereof does not exceed 10 times the hose diameter.

4. A hose clamp according to one of claims 1 to 3 **characterised in that** the radii of the rounded-off plastic body are between R20 and R100.

5. A hose clamp according to one of claims 1 to 4 **characterised in that** the plastic body terminates towards its ends at an angle of 5° to 40° relative to the longitudinal axis.

6. A hose clamp according to one of claims 1 to 5 **characterised in that** its outermost point in the closed condition is at a maximum double the hose diameter from the centre of the hose to be inserted.

7. A hose clamp according to one of claims 1 to 6 **characterised in that** gripping surfaces are cut out at the outside surface of the two arms.

8. A hose clamp according to one of claims 1 to 7 **characterised in that** the hooking mechanism is formed on the one hand by a projection facing towards the free end of the arm at the end of the hose clamp and on the other hand by a contact edge at the free end of the arm.

9. A hose clamp according to claim 8 **characterised in that** a gripping surface is provided above the projection at the free end of the hose clamp, by way of which gripping surface the elastic end of the hose clamp can be deflected out of the latching position into a release position.

## Revendications

1. Pince pour tuyau (10), de préférence pour pincer des prolongements de cathéter, constituée d'un corps en matériau synthétique en une pièce s'étendant longitudinalement avec deux bras (12, 14) reliés à un côté élastiquement l'un à l'autre, qui forment chacun une saillie (24, 26) de telle sorte que celles-ci, à l'état fermé de la pince pour tuyau, se font face, et qui à l'état fermé, peuvent être accrochés l'un dans l'autre, où des ouvertures (34, 34') sont prévues aux extrémités avant et arrière respectivement pour la réception d'un tuyau,
**caractérisée**
**en ce qu'**elle présente approximativement la forme d'un cylindre avec des cotés coupés, qui diminue des deux côtés d'une manière conique, vers ses extrémités, et en ce que les extrémités sont adaptées à la forme circulaire ronde d'un tuyau à insérer.

2. Pince pour tuyau selon la revendication 1, **caractérisée en ce que** toutes les zones d'arête sont arrondies.

3. Pince pour tuyau selon la revendication 1 ou 2, **caractérisée en ce que** sa longueur ne dépasse pas le décuple du diamètre de tuyau.

4. Pince pour tuyau selon l'une des revendications 1 à 3, **caractérisée en ce que** les rayons du corps arrondi en matériau synthétique se situent en R20 et R100.

5. Pince pour tuyau selon l'une des revendications 1 à 4, **caractérisée en ce que** le corps en matériau synthétique se termine vers ses extrémités sous un angle de 5° à 40° relativement à l'axe longitudinal.

6. Pince pour tuyau selon l'une des revendications 1 à 5, **caractérisée en ce que** son point le plus extérieur, à l'état fermé, se trouve à une distance maximale du diamètre de tuyau double depuis le milieu du tuyau à insérer.

7. Pince pour tuyau selon l'une des revendications 1 à 6, **caractérisée en ce que** des faces de prise sont évidées à la face extérieure des deux bras.

8. Pince pour tuyau selon l'une des revendications 1 à 7, **caractérisée en ce que** le mécanisme d'accrochage est formé, d'une part, par une saillie orientée vers l'extrémité libre du bras à l'extrémité de la pince pour tuyau et, d'autre part, une arête d'application réalisée à l'extrémité libre du bras.

9. Pince pour tuyau selon la revendication 8, **caractérisée en ce qu'**il est réalisé au-dessus de la saillie à l'extrémité libre de la pince pour tuyau une face de prise par laquelle l'extrémité élastique de la pince pour tuyau peut être déviée de la position d'encliquetage dans une position de libération.
